# FASCICULE DE BREVET EUROPEEN

(11) **EP 0 879 058 B1**
(45) Date de publication et mention de la délivrance du brevet: **06.06.2001**
(21) Numéro de dépôt: 97904475.7
(22) Date de dépôt: 03.02.1997
(51) Int. Cl.: A61K 35/78, A61K 31/195, A61K 31/495

(54) **MELANGES ISOLABLES A PARTIR DE GRAINES D'EUGENIA JAMBOLANA LAMARCK, LEUR PREPARATION ET L'UTILISATION DE CES MELANGES ET CERTAINS DE LEURS CONSTITUANTS COMME MEDICAMENTS**
MISCHUNGEN AUSGEHEND VON EUGENIA JAMBOLANA LAMARCK SAMEN, HERSTELLUNG UND VERWENDUNG SOLCHER MISCHUNGEN SOWIE EINIGER INHALTSSTOFFE ALS MEDIKAMENTE
MIXTURES DERIVED FROM GRAINS OF EUGENIA JAMBOLANA LAMARCK, PREPARATION AND USE OF SAID MIXTURES AND SOME OF THEIR CONSTITUENTS AS MEDICAMENTS

(30) Priorité: 06.02.1996 FR 9601389
(43) Date de publication de la demande: 25.11.1998
(73) Titulaire: Institut Malgache de Recherches Appliquées, Antananarivo 101 (MG); Aventis Pharma S.A., 92160 Antony (FR)
(72) Inventeur: RAKOTO RATSIMAMANGA, Albert, Antananarivo 101 (MG); RAKOTO RATSIMAMANGA, Suzanne, Antananarivo 101 (MG); RASOANAIVO, Philippe, Antananarivo 101 (MG); LEBOUL, Jean, F-91400 Gometz-la-Ville (FR); PROVOST, Jean, F-37260 Monts (FR); REISDORF, Daniel, F-94320 Thiais (FR)
(74) Mandataire: Savina, Jacques
(86) Numéro de dépôt international: FR9700207
(87) Numéro de publication internationale: WO9728813

(56) Documents cités:
- DE-A- 2 159 923
- FR-A- 2 465 484
- FR-M- 6 114

## Description

La présente invention concerne des mélanges isolables à partir de graines d'Eugénia Jambolana Lamarck (famille des Myrtacées), les médicaments contenant ces mélanges ou certains de leurs constituants, l'utilisation de ces mélanges et constituants pour la préparation d'un médicament antidiabétique et leur préparation.

Un extrait végétal préparé à partir de graines ou d'écorces d'Eugénia Jambolana contenant un complexe mixte polyphénolique et stérolique est décrit dans le brevet FR 2465484.

Il a maintenant été trouvé de nouveaux mélanges isolables à partir de graines d'Eugénia Jambolana Lamarck, exempts de complexe polyphénolique et stérolique, ainsi que certains constituants de ces mélanges doués de propriétés hypoglycémiantes.

Ces mélanges sont caractérisés en ce qu'ils sont exempts de dérivés polyphénoliques et stéroliques et sont isolables par broyage de graines d'Eugénia Jambolana Lamarck, macération de la poudre avec un alcool aliphatique inférieur à chaud, filtration, récupération de la partie insoluble ne contenant plus de composés polyphénoliques et stéroliques, traitement de la partie insoluble avec une solution ammoniacale, traitement du mélange ammoniacal avec un alcool aliphatique inférieur à chaud, filtration, récupération de l'insoluble et séchage de cet insoluble qui constitue le mélange I, puis éventuellement traitement du mélange I avec une solution eau-alcool aliphatique inférieur, filtration, concentration partielle du filtrat, purification sur résines adsorbantes non polaires, concentration partielle, centrifugation, ultrafiltration et isolement du mélange II.

Du mélange II peuvent également être séparés l'oxamate de sodium et les composés de formule : 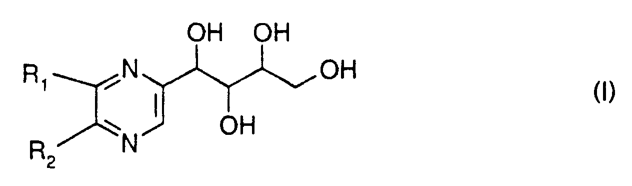 dans laquelle soit R₁ représente un atome d'hydrogène et R₂ représente une chaîne de formule :

-CH₂-CHOH-CHOH-CH₂OH (A)

-CHOH-CHOH-CHOH-CH₂OH (B)

soit R₁ représente une chaîne de formule (A) et R₂ représente un atome d'hydrogène.

L'oxamate de sodium est déjà décrit par TOUSSAINT, Ann., 120, 237 (1861).

Les composés de formule (I) sont déjà décrits par KUHN et coll., Ann., 644, 122-127 (1961); TSUCHIDA et coll., Agr. Biol. Chem., 39 (5), 1143-1148 (1975); TSUCHIDA et coll., Agr. Biol. Chem., 40 (5), 921-925 (1976); TSUCHIDA et coll., Nippon Shokuhin Kogyo Gakkaishi, 37, 154-161 (1990) et AVALOS et coll., tetrahedron, 49, 2655-2675 (1993).

La présente invention concerne également le procédé de préparation du mélange I à partir de graines d'Eugénia Jambolana Lamarck séchées et broyées finement.

La poudre est tamisée, de préférence, à l'aide d'un tamis normalisé de trous de 0,5 µm de diamètre puis subit le traitement suivant :
a - macération sous agitation dans un alcool aliphatique inférieur, à une température comprise entre 40 et 70°C,
b - filtration sous vide et récupération de l'insoluble,
c - macération sous agitation de l'insoluble avec un alcool aliphatique inférieur à une température comprise entre 40 et 70°C,
d - filtration sous vide et élimination des phases alcooliques contenant principalement les polyphénols et stérols indésirables,
e - reprise de l'insoluble dans une solution ammoniacale à une température comprise entre 10 et 30°C,
f - reprise de toute la masse humide ammoniacale dans une solution eau-alcool aliphatique inférieur, à une température comprise entre 40 et 70°C,
g - filtration et élimination de la solution alcoolique,
h - lavage de l'insoluble avec un alcool aliphatique inférieur, filtration et élimination de la solution alcoolique,
i - récupération de l'insoluble et séchage.

Dans l'étape a, on opère généralement au moyen de 2 à 10 litres d'un alcool aliphatique inférieur tel que le méthanol ou l'éthanol pour 1 kg de poudre tamisée. De préférence, on utilise 5 litres d'éthanol de titre 93-95°Gay Lussac à 60°C pendant 1 heure.

La filtration de l'étape b s'effectue de préférence sous un vide de 40kPa.

Dans l'étape c, on opère généralement au moyen de 2 à 10 litres d'un alcool aliphatique inférieur tel que le méthanol ou l'éthanol pour 1 kg de poudre tamisée de départ. De préférence, on utilise 4 litres d'éthanol de titre 93-95°Gay Lussac à une température de 60°C pendant 1 heure.

La filtration de l'étape d s'effectue de préférence sous un vide de 40 kPa.

Dans l'étape e, pour 1 kg de poudre tamisée de départ, on utilise généralement 750 à 1250 ml d'une solution aqueuse ammoniacale contenant de préférence 350 ml d'ammoniaque à 28% pour 1000 ml. Il est particulièrement avantageux d'utiliser 1 litre de la solution aqueuse ammoniacale et d'opérer pendant 10 à 30 heures et, de préférence, 20 heures à une température voisine de 20°C.

Dans l'étape f, la masse humide ammoniacale obtenue à partir de 1 kg de poudre tamisée de départ est généralement reprise, sous agitation, dans 2 à 10 litres d'un mélange alcool aliphatique inférieur-eau (méthanol ou éthanol par exemple) (70/30 à 80/20 en volumes) et, de préférence, dans 5 litres d'un mélange éthanol-eau (75/25 en volumes), à 60°C, pendant 1 heure.

Dans l'étape g, la filtration s'effectue de préférence sur une toile de coton et sous un vide d'environ 80 kPa.

Dans l'étape h, le lavage s'effectue généralement avec 500 à 1500 ml d'un alcool aliphatique inférieur (méthanol ou éthanol par exemple) pour 1 kg de poudre tamisée de départ et, de préférence, avec 1 litre d'éthanol et la filtration est réalisée sur toile de coton et sous un vide d'environ 80 kPa.

Dans l'étape i, le séchage s'effectue de préférence à l'air libre et à l'abri de la lumière.

La présente invention concerne également le procédé de préparation du mélange II.

Le mélange I obtenu précédemment est soumis aux opérations suivantes :
j - traitement du mélange I au moyen d'une solution eau-alcool aliphatique inférieur,
k - décantation puis, d'une part, soutirage de la phase supérieure qui est filtrée pour conduire au filtrat 1 et, d'autre part, traitement de la phase inférieure avec de l'eau et filtration pour conduire au filtrat 2, rassemblement des filtrats 1 et 2 et concentration à phase aqueuse,
I - traitement avec une résine adsorbante non polaire puis filtration,
m - concentration du filtrat, filtration puis ultrafiltration,
n - lyophilisation et isolement de l'extrait II.

Dans l'étape j, on utilise généralement 10 à 25 litres de la solution eau-alcool aliphatique inférieur (méthanol ou éthanol par exemple) (95/5 à 90/10 en volumes) pour 1 kg du mélange I. Il est préférable d'opérer dans 18 litres d'une solution eau-éthanol (17,7-0,93 en volumes).

Dans l'étape k, il est préférable de filtrer la phase supérieure sur toile de coton. Il est avantageux d'ajouter, pour 1 kg du mélange l, 10 à 25 litres d'eau à la phase inférieure et en particulier 10 litres et de filtrer sur fritté.

Dans l'étape k, la concentration s'effectue généralement dans un concentreur thermosiphon à une température de 35°C sous un vide de 0,4 kPa.

Dans l'étape I, on utilise de préférence la résine S861 ou des résines de type XAD commercialisées par Rhom et Haas et on filtre sur fritté.

Dans l'étape m, la concentration s'effectue généralement dans un concentrateur thermosiphon à une température de 35°C sous un vide de 0,4 kPa. Il est également avantageux d'effectuer 3 ultrafiltrations successives sur cartouche 10 kDA, 3 kDa et 1 kDa.

La présente invention concerne également le procédé de préparation de l'oxamate de sodium et des composés de formule (I).

Celui-ci consiste à soumettre le mélange Il aux opérations suivantes :
o - chromatographie du mélange II sur colonne de terre d'infusoire, récupération des fractions contenant les 4 produits et rassemblement de ces fractions en une seule fraction,
p - chromatographie de la fraction précédemment obtenue sur colonne ^{R}sephadex pour obtenir l'oxamate de sodium, le composé de formule (I) pour lequel R₁ représente un atome d'hydrogène et R₂ représente un reste (B) et un mélange du composé de formule (I) pour lequel R₁ représente un atome d'hydrogène et R₂ représente un reste (A) et du composé de formule (I) pour lequel R₁ représente un reste (A) et R₂ représente un atome d'hydrogène,
q - éventuellement chromatographie du mélange du composé de formule (I) pour lequel R₁ représente un atome d'hydrogène et R₂ représente un reste (A) et du composé de formule (I) pour lequel R₁ représente un reste (A) et R₂ représente un atome d'hydrogène par HPLC.

La chromatographie de l'étape o s'effectue au moyen d'un solvant organique comme l'heptane, l'acétate d'éthyle ou un alcool aliphatique inférieur. De préférence, on utilise une colonne ^{R}CHEM ELUT commercialisée par Prolabo saturée d'eau puis éluée successivement par de l'heptane, un mélange heptane-acétate d'éthyle (50/50 en volumes), de l'acétate d'éthyle, un mélange acétate d'éthyle-n-butanol (95/5; 90/10; 80/20; 50/50; 20/80, du n-butanol, et un mélange n-butanol-eau (98/2 puis 95/5 en volumes).

La chromatographie de l'étape p s'effectue de préférence au moyen d'un mélange eau-éthanol (50/50 en volumes).

La chromatographie de l'étape q s'effectue généralement sur colonne YMC 180DS-AQ commercialisée par AIT avec comme éluant un mélange eau à 0,1% d'acide formique.

Dans les définitions précédentes et celles qui suivent, les alcools aliphatiques inférieurs contiennent de préférence 1 à 4 atomes de carbone.

Les médicaments contenant les mélanges I ou Il ou l'oxamate de sodium ou un ou plusieurs composés de formule (I) font également partie de l'invention.

La présente invention concerne également l'utilisation des mélanges I et II, de l'oxamate de sodium et des composés de formule (I) ou un mélange de ceux-ci à la préparation de médicaments pour le traitement ou la prévention du diabète et des complications du diabète.

Les exemples suivants illustrent l'invention.

### EXEMPLE 1 : préparation du mélange I

Des graines d'Eugénia Jambolana Lamarck sont séchées à l'air libre, à l'abri de la lumière puis broyées finement. La poudre ainsi obtenue est tamisée à l'aide d'un tamis de maille 0,5 µm. 1 kg de poudre tamisée est mis à macérer sous agitation mécanique dans 5 litres d'éthanol de titre 93-95°Gay Lussac pendant une heure à 60°C. Après filtration sous vide, l'insoluble est traité de nouveau dans les mêmes conditions avec 4 litres d'éthanol de même titre à 60°C pendant encore une heure. Les deux extraits éthanoliques contenant principalement le complexe polyphénolique et stérolique indésirable sont éliminés. Après filtration complète, l'insoluble est repris avec 1 litre de solution ammoniacale (NH4OH à 28 % 350 ml et H2O distillée en quantité suffisante pour obtenir 1 litre de solution) et le mélange est laissé en contact pendant environ 20 heures à une température voisine de 20°C. La masse humide ammoniacale est remise à macérer dans 5 litres du mélange éthanol de titre 93 à 95°Gay Lussac-eau (75/25 en volumes), sous agitation mécanique pendant 1 heure à 60°C. On filtre l'insoluble et on le lave avec 1 litre d'éthanol de même titre; le filtrat et le lavage sont éliminés. L'insoluble final est séché à l'air libre et à l'abri de la lumière. On obtient ainsi le mélange I sous forme de poudre exempt de dérivés polyphénoliques et stéroliques. Le rendement à partir des graines pulvérisées et tamisées est de 80 %.

### EXEMPLE 2 - Préparation du mélange II

On agite pendant 3 heures, 1 kg du mélange I obtenu dans l'exemple 1, dans une solution contenant 17,7 litres d'eau et 0,93 litre d'éthanol. Après décantation une nuit, d'une part, la phase supérieure (13,5 litres) est soutirée puis filtrée sur toile de coton dans un filtre de 7 litres (Schott) pour donner le filtrat 1 (13,5 litres) et, d'autre part, la phase inférieure est agitée 1 heure avec 20 litres d'eau, filtrée sur fritté N°3 pour donner le filtrat 2 (24 litres). Les filtrats 1 et 2 sont rassemblés puis concentrés à phase aqueuse (33,5 litres) dans un concentreur thermosiphon (Schott) à 35°C sous vide de 0,4 kPa. Le concentrat est agité une heure avec 2,5 litres de résine S861 (Rhom et Haas) puis filtré sur fritté N°3. Le filtrat est concentré dans un concentrateur thermosiphon (Schott) à 35°C sous un vide de 0,4 kPa à 5,5 litres. Le concentrat est centrifugé sur centrifugeuse tubulaire (force centrifuge 62000g), filtré sur filtre 0,22µm (Gelman type suporcap 100) pompe. Après 3 ultrafiltrations successives sur cartouche 10 Kd, 3K d et 1 Kd et lyophilisation, on obtient 25 g du mélange Il sous forme d'une poudre hygroscopique brun foncé.

### EXEMPLE 3 - Constituants obtenus à partir du mélange II

525 mg du mélange Il obtenu dans l'exemple 2 en solution dans 1,1 ml d'eau filtrée milli Q sont chromatographiés sur une colonne de ^{R}CHEM ELUT commercialisée par PROLABO de 50 cm de hauteur et d'un diamètre de 1 cm saturée par de l'eau filtrée milli Q. L'élution est réalisée par de l'heptane avec des gradients croissants en acétate d'éthyle puis en n-butanol, en n-butanol/acide chlorhydrique et en eau (fraction 1 : heptane; fraction 2 : heptane/acétate d'éthyle (50/50 en volumes); fractions 3-4 : acétate d'éthyle; fractions 5-6 : acétate d'éthyle/n-butanol (95/5 en volumes), fractions 7-8 : acétate d'éthyle/n-butanol (90/10 en volumes), fractions 9-10 : acétate d'éthyle/n-butanol (80/20 en volumes); fractions 11-12 : acétate d'éthyle/n-butanol (50/50 en volumes), fractions 13-14 : acétate d'éthyle/n-butanol (20/80 en volumes), fractions 15-16 : n-butanol, fractions 17-18 : n-butanol/eau (98/2 en volumes), fractions 19-21 : n-butanol/eau (95/5 en volumes. Des fractions de 50 ml sont recueillies. Les fractions 19 à 21 sont réunies et concentrées à sec sous pression réduite. On obtient ainsi 108 mg d'une fraction qui est chromatographiée sur une colonne de ^{R}sephadex LH20 commercialisée par Pharmacia (hauteur 100cm, diamètre 1 cm) réalisée avec un mélange méthanol-eau (50-50 en volumes). L'élution est effectuée par le même mélange éluant; des fractions de 1 ml sont collectées.
1 - Les fractions 88 à 91 sont réunies et concentrées à sec. On obtient 14,4 mg d'un produit qui donne par cristallisation dans 0,5 ml d'éthanol 2mg de 2,5-di-(tétrahydroxybutyl)pyrazine sous forme de cristaux blancs dont les caractéristiques sont les suivantes :
   - pouvoir rotatoire [α] 20 (Na 589) = - 137° ± 2,0 (diméthylsulfoxyde; c=0,5),
   - spectre infra-rouge réalisé sur un appareil Nicolet 60SX-R en solution dans le KBr, principales bandes d'absorption caractéristiques : 3281 cm-1 (ν OH liés dont H2O), 2972+2940+2901+2880 cm-1 (ν CH des CH2 et CHOH), 2733 cm-1 (ν OH liés), 1635cm-1 (déformations des OH, dont H2O),1491cm-1 (ν C=C et C=N du noyau pyrazine), 1449 cm-1 (ν C=C et C=N du noyau pyrazine + dé-formations des OH), 1413 cm-1 (déformations des OH), 1343 cm-1 (ν C=C et C=N du noyau pyrazine), 1309+1290+1251+1215+1181+1161+1123 cm-1 (déformations des CH), 1092 cm-1 (ν CO des alcools secondaires), 1048+1035 cm-1 (ν CO des alcools primaires + noyau pyrazine), 947+899+854 cm-1 (alcools secondaires), 877 cm-1 (νCH du noyau pyrazine), 727 cm-1 (noyau pyrazine + déformations des OH), 639 cm-1 (noyau pyrazine), 607+531cm-1 larges (déformations des OH), 451+411 cm-1 (noyau pyrazine),
   - spectre de masse réalisé sur un appareil FINNIGAN TSQ46, mode d'ionisation masse M/z=321 (MH)+,
   - spectre de R.M.N.1H (600 MHz, DMSO, déplacement chimique en ppm) : 3,40 et 3,61 (2mts, 2H chacun : CH2 en 4', 4"); 3,58 (2 mts, 2H chacun : CH en 2',2",3',3"): 4,36 (t large, 2H : OH en 4',4"); 4,40 (d, J=7,2 Hz, 2H : OH en 2', 2"); 4,63 (d, J=4,8 Hz, 2H : OH en 3',3"); 4,95 (dd J=6,0 et 0,6 Hz, 2H : CH en 1', 1"); 5,30 (d, J=6,0 Hz, 2H : OH en 1', 1"); 8,61 (s, 2H, CH en 3 et 6),
   - spectre ultra violet : λ max = 275 nm (ε=8260); 206 nm (ε=10220) (c=19 mg/ml; eau), λ max = 276 nm (ε=7960); 206 nm (ε=9920) (c=19 mg/ml; HCI 0,1N), λ max = 275 nm (ε=7690); (c=19 mg/ml; KOH 0,1N),
   - HPLC sur colonne Y.M.C. 18ODS-AQ de 150x4,6 mm (lot AIT/DE940377) commercialisée par AIT, élution isocratique H₂O +0,1% d'acide formique avec un débit de 1ml/mn, détection UV à 270 nm, temps de rétention : 2 mn 32 s.
2 - Les fractions 92 et 93 sont réunies et concentrées à sec. On obtient 9 mg d'une fraction qui donne par cristallisation dans 0,5 ml d'éthanol 1,2 mg d'oxamate de sodium présentant les mêmes caractéristiques que celles décrites par TOUSSAINT, Ann., 120, 237 (1861).
3 - Les fractions 94 à 97 sont réunies et concentrées à sec. On obtient 25,9 mg d'une fraction qui donne par cristallisation dans 0,5 ml d'éthanol 6,2 mg d'un mélange de 2-(tétrahydroxybutyl)-5-(2',3',4'-trihydroxybutyl)pyrazine et 2-(tétrahydroxybutyl)-6-(2',3',4'-trihydroxybutyl)pyrazine qui est à nouveau séparé par HPLC sur colonne Y.M.C. 18ODS-AQ de 150x4,6 mm (lot AIT/DE940377); élution isocratique H₂O +0,1% d'acide formique avec un débit de 1ml/mn, détection UV à 270 nm.

On obtient ainsi 5,2 mg de 2-(tétrahydroxybutyl)-5-(2',3',4'-trihydroxybutyl)pyrazine qui présente les caractéristiques suivantes :
- pouvoir rotatoire [α]²⁰ (Na 589) = - 116,3° ± 1,7 (diméthylsulfoxyde; c=0,5),
- spectre infra-rouge réalisé sur un appareil Nicolet 60SX-R en solution dans le KBr, principales bandes d'absorption caractéristiques à 3398 cm-1 (ν OH liés dont H2O), 2951+2922+2891 cm-1 (ν CH des CHOH), 2761 cm-1 (ν OH liés), 1636cm-1 (déformations des OH , dont H2O), 1483+1463cm-1 (ν C=C et C=N du noyau pyrazine), 1411 cm-1 (déformation des OH), 1367+1328+1270+1227+1191 cm -1 (déformations des CH), 1071 cm-1 (ν CO des alcools secondaires), 1041cm-1 (ν CO des alcools primaires + noyau pyrazine), 943+897 cm-1 (alcools secondaires), 869 cm-1 (ν CH du noyau pyrazine), 645 cm-1 (CH du noyau pyrazine), 607cm-1 larges (déformations des OH), 446+409 cm-1 (noyau pyrazine),
- spectre de masse réalisé sur un appareil FINNIGAN TSQ46, mode d'ionisation masse M/z=305 (MH)+,
- spectre de R.M.N.1H (600 MHz, DMSO, déplacement chimique en ppm) : 2,70 et 3,04 (2 dd, J=9,0 et 15,0 Hz et J=3,0 et 15,0 Hz, 1H chacun : CH2 en 1"); entre 3,30 et 3,45 (mts, CH en 3", 4', 4"); entre 3,53 et 3,65 (mts, 4H : CH en 2',3',4',4"), 3,73 (mt, 1H : CH en 2"); 4,37 (t large, 1H : OH en 4'); 4,42 (mt, 2H : OH en 2' et 4"); 4,60 (d, J=6 Hz, 1H : OH en 2"); 4,63 (d, J=6 Hz, 1H : OH en 3'); 4,67 (d, J=6 Hz, 1H : OH en 3"); 4,92 (dd J=6,0 et 0,6 Hz, 1H : CH en 1'); 5,30 (d, J=6,0 Hz, 1H : OH en 1'); 8,39 (s, 1H : CH en 6); 8,61 (s, 1H : CH en 3),
- spectre ultra violet : λ max = 276 nm (ε=7756) ; 206 nm (ε=8738) (c=19 mg/ml; eau), λ max = 277 nm (ε=7218); 208 nm (ε=7171 ) (c=19 mg/ml; HCI 0,1 N), λ max = 276 nm (ε=7467) (c=19 mg/ml; KOH 0,1N),
- HPLC sur colonne Y.M.C. 18ODS-AQ de 150x4,6 mm (lot AIT/DE940377) commercialisée par AIT, élution isocratique H₂O + 0,1% d'acide formique avec un débit de 1ml/mn, détection UV à 270 nm, temps de rétention : 3mn 75 s
et 1 mg de 2-(tétrahydroxybutyl)-6-(2',3',4'-trihydroxybutyl)pyrazine qui présente les caractéristiques suivantes :
- spectre de R.M.N.1H (600 MHz, DMSO, déplacement chimique en ppm) : 2,70 et 3,04 (2 dd, J=9,0 et 15,0 Hz et J=3,0 et 15,0 Hz, 1H chacun : CH2 en 1"); entre 3,30 et 3,45 (mts, CH en 3", 4', 4"); entre 3,53 et 3,65 (mts, 4H : CH en 2',3',4',4"), 3,73 (mt, 1H : CH en 2"); 4,37 (t large, 1H : OH en 4'); 4,42 (mt, 2H : OH en 2' et 4"); 4,60 (d, J=6 Hz, 1H : OH en 2"); 4,63 (d, J=6 Hz, 1H : OH en 3'); 4,67 (d, J=6 Hz, 1H : OH en 3"); 4,92 (dd J=6,0 et 0,6 Hz, 1H : CH en 1'); 5,30 (d, J=6,0 Hz : 1H, OH en 1'); 8,31 (s, 1H : CH en 5); 8,53 (s, 1H : CH en 3),
- HPLC sur colonne Y.M.C. 18ODS-AQ de 150x4.6 mm (lot AIT/DE940377) commercialisée par AIT, élution isocratique H₂O +0,1% d'acide formique avec un débit de 1ml/mn, détection UV à 270 nm, temps de rétention : 3 mn 61 s.

L'activité hypoglycémiante des mélanges I et II, de l'oxamate de sodium et des composés de formule (I) a été déterminée chez la souris rendue diabétique par la streptozocine et chez la souris normale en hyperglycémie post-prandiale selon les protocoles suivants :

### I - Souris rendues diabétiques par la streptozotocine

Des souris Swiss albinos pesant entre 22 et 25 g sont rendues diabétiques par la streptozotocine administrée par injection intrapéritonéale à la dose de 210 ou 265 mg/Kg de souris, diluée dans un tampon citrate à une concentration telle que chaque souris reçoive 0,2 ml de la solution au jour 1 (J1). 3 à 4 jours plus tard, on contrôle sur 2 à 3 souris si le diabète est installé (glycémies supérieures à 7,2 mmole/litre (130 mg pour 100 ml). On mesure alors la glycémie après un jeûne de 4 heures. Si les souris sont devenues diabétiques on les répartit en lots de 5 à 7 souris. Chacun des lots reçoit à partir de J1 et quotidiennement une dose sélectionnée de produit. L'administration se fait une fois par jour et à heure fixe, par tubage gastrique et sous un volume de 0,4 ml d'eau distillée comme véhicule. On constitue deux lots de témoins :
- un lot de souris diabétiques non traitées
- un lot de souris normales
Ces deux lots de témoins reçoivent par intubation gastrique et simultanément aux souris traitées, 0,4 ml de véhicule.
Le traitement dure 4 jours. Le 5ème jour (J5) il n'y a pas d'administration de produit. Après un jeûne de 4 heures les glycémies finales sont mesurées.

### Il - Souris normales en hyperglycémie post-prandiale

Des souris Swiss albinos pesant entre 22 et 25 g sont mises en hyperglycémie post-prandiale par le protocole suivant :
- Jeûne 2 heures
- nourriture en excès pendant 1 heure
- Jeûne 2 heures
On mesure la glycémie à la fin des deux dernières heures de jeûne, qui constitue la glycémie au temps T0. Les souris sont alors réparties en lots homogènes en fonction des glycémies mesurées. Les produits à évaluer sont administrés sans délai par intubation gastrique dans 0,4 ml d'eau distillée. Le lot témoin reçoit l'excipient (0,4 ml d'eau distillée). Après une heure, les glycémies finales sont mesurées qui constituent la glycémie au temps T60.

| Résultats obtenus sur la glycémie de la souris rendue diabétique par la streptozotocine | | | | |
|---|---|---|---|---|
| PRODUITS mg/souris/jour | NOMBRE DE SOURIS | GLYCEMIE A J1 mg/100ml | GLYCEMIE A J5 mg/100ml | % inhibition |
| Oxamate de sodium (0,5mg) | 5 | 153,40±11,53 | 89,90±13,94 | -41,46 |
| Mélange II (0.5mg) | 5 | 270,50±58,72 | 117,50±17,35 | -56,56 |
| 2-(tétrahydroxybutyl)-5-(2',3',4'-trihydroxybutyl) pyrazine (0,5mg) | 5 | 304,25±99,35 | 164,50±95,13 | -45,93 |
| 2-(tétrahydroxybutyl)-5-(2',3',4'-trihydroxybutyl) pyrazine et 2-(tétrahydroxybutyl)-6-(2',3',4'-trihydroxybutyl)pyrazine (50/50) (0,25mg) | 6 | 320,83±130,30 | 174,00±97,74 | -45,77 |
| Témoins | 5 | 221,66±50,17 | 210.33 ±20,07 | -5,11 |

| Résultats obtenus sur la glycémie de la souris normale en hyperglycémie post - prandiale | | | | |
|---|---|---|---|---|
| PRODUITS mg/souris/jour | NOMBRE DE SOURIS | GLYCEMIE A TO mg/100ml | GLYCEMIE T60 A mg/100ml | % inhibition |
| Mélange I (5mg) | 5 | 129,50±29,06 | 87,60±14,16 | -32,35 |
| Oxamate de sodium (0,5mg) | 15 | 136,46±23.69 | 102,93±20.95 | -24,57 |
| Mélange II (0,5mg) | 15 | 133,93±17,50 | 105,60±16,91 | -21,15 |
| 2,5-di(tétrahydroxybutyl) pyrazine (0,5 mg) | 10 | 136,25 ±16,92 | 102,41 ±14,12 | -24,83 |
| 2-(tétrahydroxybutyl)-5-(2',3',4'-trihydroxybutyl) pyrazine (0,5mg) | 10 | 128,75±21,23 | 90,50 ±19,55 | -29,70 |
| 2-(tétrahydroxybutyl)-5-(2',3',4'-trihydroxybutyl) pyrazine et 2-(tétrahydroxybutyl)-6-(2,3',4-trihydroxybutyl)pyrazine (50/50) (0.25mg) | 5 | 126,60 ±17,03 | 100,60 ±16,88 | -20,53 |
| Témoins | 10 | 137,83±13,99 | 130,50±20,23 | -5,32 |

Les mélanges selon l'invention, l'oxamate de sodium et les composés de formule (I)présentent une faible toxicité. Leur DL50 est supérieure à 2000 mg/kg par voie orale chez la souris.

Les mélanges selon l'invention, l'oxamate de sodium et les composés de formule (I) abaissent la glycémie chez le sujet diabètique et sont donc utiles dans le traitement du diabète et les complications du diabète.

Lorsque ces produits sont utilisés en monothérapie dans le traitement du diabète, il n'y a pas de risque d'hypoglycémie. Ce sont des antidiabétiques vrais. Il semble que cet effet résulte d'une augmentation périphérique du glucose. Les produits ne stimulent pas significativement la sécrétion d'insuline mais la présence de petites quantités d'insuline est nécessaire à leur action.

Chez le rat des sables (Psammomys obesus) spontanément diabétique en captivité les produits réduisent l'hyperglycémie, préviennent ou réduisent la cataracte et ramènent un certain degré de fécondité.

En thérapeutique humaine, ces produits sont donc utiles dans la prévention et le traitement du diabète et notamment du diabète de type Il (NID diabète) ne présentant pas d'acétonurie, du diabète de l'obèse, du diabète de la cinquantaine, du diabète métapléthorique, du diabète du sujet âgé et du diabète léger. Ils peuvent être utilisés en complément de l'insulinothérapie (en raison de leur activité potentialisatrice de l'insuline) dans le diabète insulino dépendant où ils permettent de diminuer progressivement la dose d'insuline, le diabète instable, le diabète insulinorésistant, en complément des sulfamides hypoglycémiants quand ceux-ci ne déterminent pas de baisse suffisante de la glycémie. Ces produits peuvent être utilisés également dans les complications du diabète telles que les hyperlipémies, les troubles du métabolisme lipidique, les dyslipémies, l'obésité. Ils sont aussi utiles dans la prévention et le traitement des lésions d'athérosclérose et leurs complications (coronopathies, infarctus du myocarde, cardiomyopathies, évolution de ces trois complications vers l'insuffisance ventriculaire gauche, artériopathies diverses, artérites des membres inférieurs avec claudication et évolution vers les ulcères et la gangrène, insuffisance vasculaire cérébrale et ses complications, impuissance sexuelle d'origine vasculaire), la rétinopathie diabétique et de toutes ses manifestations (augmentation de la perméabilité capillaire, dilatation et thrombose capillaire, microanévrismes, shunt artérioveineux, dilatation veineuse, hémorragies ponctiformes et maculaires, exudats, oedèmes maculaires, manifestations de la rétinopathie proliférante : néovaisseaux, cicatrices de rétinite proliférante, hémorragies du vitrée, décollement de la rétine), la cataracte diabétique, la neuropathie diabétique dans ses diverses formes (polyneuropathies périphériques et ses manifestations telles que paresthésies, hyperesthésies et douleurs, mononeuropathies, radiculopathies, neuropathies autonomes, amyotrophies diabétiques), les manifestations du pied diabétique (ulcères des extrémités inférieures et du pied), la néphropathie diabétique dans ses deux formes diffuse et nodulaire, l'athéromatose (élévation des HDL lipoproteïnes favorisant l'élimination du cholestrérol à partir des plaques d'athérome, baisse des LDL lipoproteïnes, baisse du rapport LDL/HDL, inhibition de l'oxydation des LDL, diminution de l'adhésivité plaquettaire), des hyperlipémies et des dyslipémies (hypercholestérolémies, hypertriglycéridémies, normalisation du taux des acides gras, normalisation de l'uricémie, normalisation des apoprotéïnes A et B), de la cataracte, de l'hypertension artérielle et ses conséquences.

Les médicaments selon l'invention sont constitués par un mélange selon l'invention, l'oxamate de sodium, un composé de formule (I) ou une combinaison de ces produits, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

Les doses dépendent de l'effet recherché, de la durée du traitement et de la voie d'administration utilisée; elles sont généralement comprises entre 150 mg et 600 mg par jour par voie orale pour un adulte avec des doses unitaires allant de 50 mg à 200 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- Produit actif 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Produit actif 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 50 mg de produit actif ayant la composition suivante :
- Produit actif 50 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Mélanges exempts de dérivés polyphénoliques et stéroliques et isolables par broyage de graines d'Eugénia Jambolana Lamarck, macération de la poudre avec un alcool aliphatique inférieur à chaud, filtration, récupération de la partie insoluble ne contenant plus de composés polyphénoliques et stéroliques, traitement de la partie insoluble avec une solution ammoniacale, traitement du mélange ammoniacal avec un alcool aliphatique inférieur à chaud, filtration, récupération de l'insoluble et séchage de cet insoluble qui constitue le mélange I et éventuellement traitement du mélange I avec une solution eau-alcool aliphatique inférieur, filtration, concentration partielle du filtrat, purification sur résines adsorbantes non polaires, concentration partielle, centrifugation, ultrafiltration et isolation du mélange II.

2. Procédé de préparation du mélange I selon la revendication 1 caractérisé en ce que des graines d'Eugénia Jambolana Lamarck sont séchées, broyées finement, tamisées et la poudre obtenue subit le traitement suivant :
a - macération sous agitation dans un alcool aliphatique inférieur (1-4C), à une température comprise entre 40 et 70°C,
b - filtration sous vide et récupération de l'insoluble,
c - macération sous agitation de l'insoluble avec un alcool aliphatique inférieur (1-4C) à une température comprise entre 40 et 70°C,
d - filtration sous vide et élimination des phases alcooliques contenant principalement les polyphénols et stérols indésirables,
e - reprise de l'insoluble dans une solution ammoniacale à une température comprise entre 10 et 30°C,
f - reprise de toute la masse humide ammoniacale dans une solution eau-alcool aliphatique inférieur (1-4C), à une température comprise entre 40 et 70°C,
g - filtration et élimination de la solution alcoolique,
h - lavage de l'insoluble avec un alcool aliphatique inférieur (1-4C), filtration et élimination de la solution alcoolique,
i - récupération de l'insoluble et séchage du mélange I.

3. Procédé selon la revendication 2 pour lequel, dans l'étape a, on opère au moyen de 2 à 10 litres d'un alcool aliphatique inférieur pour 1 kg de poudre tamisée.

4. Procédé selon l'une des revendications 2 ou 3 pour lequel, dans l'étape a on utilise 5 litres d'éthanol de titre 93-95°Gay Lussac à 60°C pendant 1 heure.

5. Procédé selon l'une des revendications 2 à 4 pour lequel la filtration des étapes b et d s'effectuent sous un vide de 40 kPa.

6. Procédé selon l'une des revendications 2 à 5 pour lequel dans l'étape c, on opère au moyen de 2 à 10 litres d'un alcool aliphatique inférieur pour 1 kg de poudre tamisée de départ.

7. Procédé selon l'une des revendications 2 à 6 pour lequel, dans l'étape c, on utilise 4 litres d'éthanol de titre 93-95°Gay Lussac, à une température de 60°C pendant 1 heure.

8. Procédé selon l'une des revendications 2 à 7 pour lequel dans l'étape e, pour 1 kg de poudre tamisée de départ, on utilise 750 à 1250 ml d'une solution aqueuse ammoniacale.

9. Procédé selon l'une des revendications 2 à 8 pour lequel dans l'étape e, pour 1 kg de poudre tamisée de départ on utilise 1 litre de solution aqueuse ammoniacale contenant 350 ml d'ammoniaque à 28% et on opère à une température voisine de 20°C pendant 10 à 30 heures heures.

10. Procédé selon l'une des revendications 2 à 9 pour lequel dans l'étape f, la masse humide ammoniacale obtenue à partir de 1 kg de poudre tamisée de départ est reprise dans 2 à 10 litres d'une solution eau-alcool aliphatique inférieur.

11. Procédé selon l'une des revendications 2 à 10 pour lequel dans l'étape f on opère dans 5 litres d'un mélange éthanol-eau (75/25 en volumes), à 60°C, pendant 1 heure.

12. Procédé selon l'une des revendications 2 à 11 pour lequel dans l'étape h, le lavage s'effectue avec 500 à 1500 ml d'un alcool aliphatique inférieur pour 1 kg de poudre tamisée de départ.

13. Procédé selon l'une des revendications 2 à 12 pour lequel dans l'étape h le lavage s'effectue avec 1 litre d'éthanol et la filtration est réalisée sur toile de coton et sous un vide d'environ 80 kPa.

14. Procédé selon l'une des revendications 2 à 12 pour lequel dans l'étape i, le séchage s'effectue à l'air libre et à l'abri de la lumière.

15. Procédé de préparation du mélange II selon la revendication 1 pour lequel le mélange I selon la revendication 1 est soumis aux opérations suivantes :
j - traitement du mélange I selon la revendication 1 au moyen d'une solution eau-alcool aliphatique inférieur (1-4C),
k - décantation puis, d'une part, soutirage de la phase supérieure qui est filtrée pour conduire au filtrat 1 et, d'autre part, traitement de la phase inférieure avec de l'eau et filtration pour conduire au filtrat 2, rassemblement des filtrats 1 et 2 et concentration à phase aqueuse,
l - traitement avec une résine adsorbante non polaire puis filtration,
m - concentration du filtrat, filtration puis ultrafiltration,
n - lyophilisation et isolation de l'extrait II.

16. Procédé selon la revendication 15 pour lequel dans l'étape j, on utilise 10 à 25 litres de la solution eau-alcool aliphatique inférieur (95/5 à 90/10 en volumes) pour 1 kg du mélange I.

17. Procédé selon l'une des revendications 15 ou 16 pour lequel dans l'étape j on utilise 18 litres d'une solution eau-éthanol (17,7-0,93 en volumes).

18. Procédé selon l'une des revendications 15 à 17 pour lequel dans l'étape k, on filtre la phase supérieure sur toile de coton et on ajoute, pour 1 kg du mélange I, 10 à 25 litres d'eau à la phase inférieure et on filtre sur fritté.

19. Procédé selon l'une des revendications 15 à 18 pour lequel dans l'étape k, la concentration s'effectue généralement dans un concentreur thermosiphon à une température de 35°C sous un vide de 0,4 kPa.

20. Procédé selon l'une des revendications 15 à 19 pour lequel dans l'étape I, on utilise la résine S861 ou des résines de type XAD commercialisées par Rhom et Haas et on filtre sur fritté.

21. Procédé selon l'une des revendications 15 à 20 pour lequel dans l'étape m, la concentration s'effectue dans un concentrateur thermosiphon à une température de 35°C sous un vide de 0,4 kPa.

22. Procédé selon l'une des revendications 15 à 21 pour lequel dans l'étape m on effectue 3 ultrafiltrations successives sur cartouche 10 kDa, 3 kDa et 1 kDa.

23. Procédé de préparation de l'oxamate de sodium et des composés de formule : 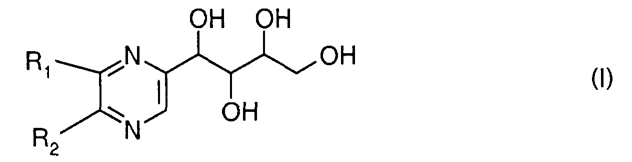 dans laquelle soit R₁ représente un atome d'hydrogène et R₂ représente une chaîne de formule :
-CH₂-CHOH-CHOH-CH₂OH (A)
-CHOH-CHOH-CHOH-CH₂OH (B)
soit R₁ représente une chaîne de formule (A) et R₂ représente un atome d'hydrogène pour lequel le mélange Il selon la revendication 1 est soumis aux opérations suivantes :
o - chromatographie du mélange II selon l'invention sur colonne de terre d'infusoire, récupération des fractions contenant les 4 produits et rassemblement de ces fractions en une seule fraction,
p - chromatographie de la fraction précédemment obtenue sur colonne ^{R}sephadex pour obtenir l'oxamate de sodium, le composé de formule (I) pour lequel R₁ représente un atome d'hydrogène et R₂ représente un reste (B) et un mélange du composé de formule (I) pour lequel R₁ représente un atome d'hydrogène et R₂ représente un reste (A) et du composé de formule (I) pour lequel R₁ représente un reste (A) et R₂ représente un atome d'hydrogène,
q - éventuellement chromatographie du mélange du composé de formule (I) pour lequel R₁ représente un atome d'hydrogène et R₂ représente un reste (A) et du composé de formule (I) pour lequel R₁ représente un reste (A) et R₂ représente un atome d'hydrogène par HPLC.

24. Procédé selon la revendication 23 pour lequel la chromatographie de l'étape o est effectuée sur colonne de ^{R}CHEM ELUT commercialisée par Prolabo saturée d'eau puis éluée successivement par de l'heptane, un mélange heptane-acétate d'éthyle (50/50 en volumes), un mélange acétate d'éthyle-n-butanol (95/5; 90/10; 80/20; 50/50; 20/80 puis 0/100 en volumes) et un mélange n-butanol-eau (98/2 puis 95/5 en volumes)..

25. Procédé selon la revendication 24 pour lequel la chromatographie de l'étape p s'effectue au moyen d'un mélange eau-éthanol (50/50 en volumes).

26. Procédé selon la revendication 24 pour lequel la chromatographie de l'étape q s'effectue sur colonne YMC 180DS-AQ commercialisée par AIT avec comme éluant un mélange eau à 0,1 % d'acide formique.

27. Médicaments caractérisés en ce qu'ils contiennent en tant que principe actif un mélange I selon la revendication 1 ou un mélange II selon la revendication 1 ou l'oxamate de sodium ou un ou plusieurs composés de formule : 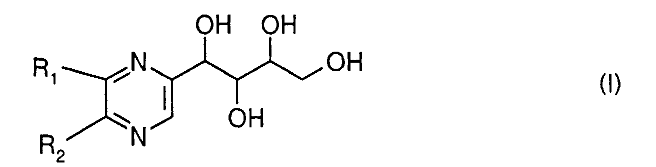 dans laquelle soit R₁ représente un atome d'hydrogène et R₂ représente une chaîne de formule:
-CH₂-CHOH-CHOH-CH₂OH (A)
-CHOH-CHOH-CHOH-CH₂OH (B)
soit R₁ représente une chaîne de formule (A) et R₂ représente un atome d'hydrogène et un ou plusieurs excipients.

28. Utilisation d'un mélange I selon la revendication 1 ou un mélange II selon la revendication 1 ou l'oxamate de sodium ou un ou plusieurs composés de formule : 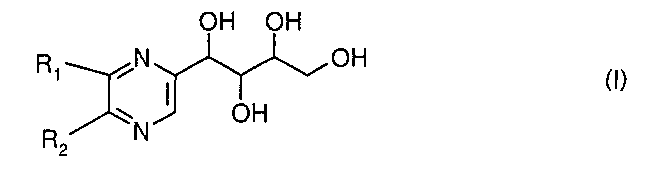 dans laquelle soit R₁ représente un atome d'hydrogène et R₂ représente une chaîne de formule :
-CH₂-CHOH-CHOH-CH₂OH (A)
-CHOH-CHOH-CHOH-CH₂OH (B)
soit R₁ représente une chaîne de formule (A) et R₂ représente un atome d'hydrogène pour la préparation d'un médicament pour la prévention et le traitement du diabète et des complications du diabète.

## Patentansprüche

1. Gemische, ausgenommen Polyphenol-Sterol-Derivate, und isolierbar durch Zerkleinerung von Körnern von Eugenia Jambolana Lamarck, Mazeration des Pulvers mit einem niedrigen aliphatischen Alkohol in der Hitze, Filtration, Isolierung des unlöslichen Teils, der nicht mehr die polyphenolischen und sterolischen Verbindungen enthält, Behandlung des unlöslichen Teils mit einer ammoniakalischen Lösung, Behandlung des ammoniakalischen Gemisches mit einem niedrigen aliphatischen Alkohol in der Wärme, Filtration, Isolierung des unlöslichen Rückstands und Trocknen dieses unlöslichen Rückstands, der das Gemisch I darstellt, und gegebenenfalls Behandlung des Gemisches I mit einer Lösung aus Wasser und einem niedrigen aliphatischen Alkohol, Filtration, Teilkonzentration des Filtrats, Reinigung über adsorbierende nichtpolare Harze, Teilkonzentration, Zentrifugation, Ultrafiltration und Isolierung des Gemisches II.

2. Verfahren zur Herstellung des Gemisches I nach Anspruch 1, dadurch **gekennzeichnet**, dass die Körner von Eugenia Jambolana Lamarck getrocknet, fein zerkleinert, gesiebt werden und dass das so erhaltene Pulver die folgende Behandlung erhält:
a - Mazeration unter Rühren in einem niedrigen aliphatischen Alkohol (C1-4) bei einer Temperatur zwischen 40 und 70°C,
b - Filtration im Vakuum und Gewinnung des unlöslichen Rückstands,
c - Mazeration unter Rühren des unlöslichen Rückstands mit einem niedrigen aliphatischen Alkohol (C1-4) bei einer Temperatur zwischen 40 und 70°C,
d - Filtration im Vakuum und Abziehen der alkoholischen Phasen, die hauptsächlich die unerwünschten Polyphenole und Sterole enthalten,
e - Wiederaufnahme des unlöslichen Rückstands in einer ammoniakalischen Lösung bei einer Temperatur zwischen 10 und 30°C,
f - Aufnahme der gesamten feuchten ammoniakalischen Masse in eine Lösung aus Wasser und einem niedrigem aliphatischem Alkohol (C1-4) bei einer Temperatur zwischen 40 und 70°C,
g - Filtration und Abziehen der alkoholischen Lösung,
h - Waschen des unlöslichen Rückstands mit einem niedrigen aliphatischen Alkohol (C1-4), Filtration und Abziehen der alkoholischen Lösung,
i - Wiedergewinnen des unlöslichen Rückstands und Trocknen des Gemisches I.

3. Verfahren nach Anspruch 2, wobei man in der Stufe a mit 2 bis 10 Litern eines niedrigen aliphatischen Alkohols auf 1 kg gesiebtes Pulver arbeitet.

4. Verfahren nach einem der Ansprüche 2 oder 3, wobei man in der Stufe a 5 Liter Ethanol mit einem Gehalt von 93 bis 95° Gay Lussac bei 60°C während 1 Stunde verwendet.

5. Verfahren nach einem der Ansprüche 2 bis 4, wobei man die Filtration der Stufen b und d in einem Vakuum von 40 kPa durchführt.

6. Verfahren nach einem der Ansprüche 2 bis 5, wobei man in Stufe c mit 2 bis 10 Litern eines niedrigen aliphatischen Alkohols auf 1 kg gesiebtes Ausgangspulver arbeitet.

7. Verfahren nach einem der Ansprüche 2 bis 6, wobei man in Stufe c 4 Liter Ethanol mit einem Gehalt von 93 bis 95° Gay Lussac bei einer Temperatur von 60°C während 1 Stunde verwendet.

8. Verfahren nach einem der Ansprüche 2 bis 7, wobei man in Stufe e auf 1 kg gesiebtes Ausgangspulver 750 bis 1250 ml einer wässrigen ammoniakalischen Lösung verwendet.

9. Verfahren nach einem der Ansprüche 2 bis 8, wobei man in Stufe e auf 1 kg gesiebtes Ausgangspulver 1 Liter wässriger ammoniakalischer Lösung, die 350 ml 28%igen Ammoniak enthält, verwendet und bei einer Temperatur bei 20°C während 10 bis 30 Stunden arbeitet.

10. Verfahren nach einem der Ansprüche 2 bis 9, wobei man in Stufe f die aus 1 kg gesiebtem Ausgangspulver erhaltene feuchte ammoniakalische Masse in 2 bis 10 Litern einer Lösung aus Wasser und einem niedrigen aliphatischen Alkohol aufnimmt.

11. Verfahren nach einem der Ansprüche 2 bis 10, wobei man in Stufe f in 5 Litern eines Gemisches aus Ethanol-Wasser (75/25 in Volumina) bei 60°C während 1 Stunde arbeitet.

12. Verfahren nach einem der Ansprüche 2 bis 11, wobei man in Stufe h das Waschen mit 500 bis 1500 ml eines niedrigen aliphatischen Alkohols auf 1 kg gesiebtes Ausgangspulver durchführt.

13. Verfahren nach einem der Ansprüche 2 bis 12, wobei man in Stufe h das Waschen mit 1 Liter Ethanol durchführt und die Filtration über ein Baumwolltuch und im Vakuum von etwa 80 kPa durchführt.

14. Verfahren nach einem der Ansprüche 2 bis 12, wobei man in Stufe i an freier Luft und ohne Licht trocknet.

15. Verfahren zur Herstellung des Gemisches II nach Anspruch 1, wobei das Gemisch I nach Anspruch 1 den folgenden Arbeitsschritten unterworfen wird:
j - Behandlung des Gemisches I nach Anspruch 1 mit einer Lösung aus Wasser und einem niedrigen aliphatischen Alkohol (C1-4),
k - Dekantieren, dann einerseits Umfüllen der oberen Phase, die filtriert wird, wobei Filtrat 1 erhalten wird, und andererseits Behandlung der unteren Phase mit Wasser und Filtration, wodurch Filtrat 2 erhalten wird, Vereinigung der Filtrate 1 und 2 und Konzentrieren auf die wässrige Phase,
l - Behandeln mit einem nichtpolaren adsorbierenden Harz, dann Filtration,
m - Konzentrieren des Filtrats, Filtration, dann Ultrafiltration,
n - Lyophilisation und Isolieren des Extrakts II.

16. Verfahren nach Anspruch 15, worin man in Stufe j 10 bis 25 Liter der Lösung aus Wasser und einem niedrigen aliphatischen Alkohol (95/5 bis 90/10 in Volumina) auf 1 kg des Gemisches I verwendet.

17. Verfahren nach einem der Ansprüche 15 oder 16, wobei man in Stufe j 18 Liter einer Lösung aus Wasser-Ethanol (17,7 bis 0,93 in Volumina) verwendet.

18. Verfahren nach einem der Ansprüche 15 bis 17, wobei man in Stufe k die obere Phase über ein Baumwolltuch filtriert und auf 1 kg des Gemisches I 10 bis 25 Liter Wasser der unteren Phase zusetzt und über eine Fritte filtriert.

19. Verfahren nach einem der Ansprüche 15 bis 18, wobei man in Stufe k im Allgemeinen in einem Thermosiphon-Konzentrator bei einer Temperatur von 35°C unter einem Vakuum von 0,4 kPa einengt.

20. Verfahren nach einem der Ansprüche 15 bis 19, wobei man in Stufe Lösung das Harz S861 oder Harze vom Typ XAD, die von Röhm & Haas vertrieben werden, verwendet und über eine Fritte filtriert.

21. Verfahren nach einem der Ansprüche 15 bis 20, wobei man in Stufe m die Konzentrierung mit einem Thermosiphon-Konzentrator bei einer Temperatur von 35°C in einem Vakuum von 0,4 kPa durchführt.

22. Verfahren nach einem der Ansprüche 15 bis 21, wobei man in Stufe m 3 aufeinanderfolgende Ultrafiltrationen über eine Kartusche 10 kDa, 3 kDa und 1 kDa durchführt.

23. Verfahren zur Herstellung von Natriumoxamat und Verbindungen der Formel: 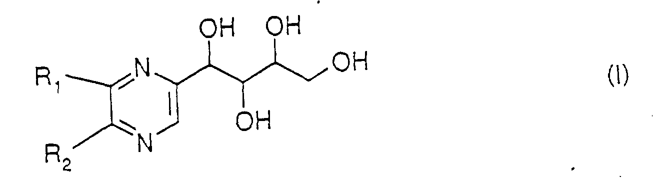 worin entweder R₁ ein Wasserstoffatom bedeutet und R₂ eine Kette der Formel:
-CH₂-CHOH-CHOH-CH₂OH (A)
-CHOH-CHOH-CHOH-CH₂OH (B)
bedeutet oder R₁ eine Kette der Formel (A) bedeutet und R₂ ein Wasserstoffatom bedeutet, wobei das Gemisch II nach Anspruch 1 den folgenden Arbeitsschritten unterworfen wird:
o - Chromatographie des erfindungsgemäßen Gemisches II über eine Säule mit Infusorienerde, Wiedergewinnung der Fraktionen, die die 4 Produkte enthalten, und Vereinigung dieser Fraktionen zu einer einzigen Fraktion,
p - Chromatographie der vorstehend erhaltenen Fraktion über eine ^{R}Sephadex-Säule zum Erhalt von Natriumoxamat, der Verbindung der Formel (I), worin R₁ ein Wasserstoffatom bedeutet und R₂ einen Rest (B) bedeutet und eines Gemisches der Verbindung der Formel (I), worin R₁ ein Wasserstoffatom bedeutet und R₂ einen Rest (A) bedeutet, und der Verbindung der Formel (I), worin R₁ einen Rest (A) bedeutet und R² ein Wasserstoffatom bedeutet,
q - gegebenenfalls Chromatographie des Gemisches der Verbindung der Formel (I), worin R₁ ein Wasserstoffatom bedeutet und R₂ einen Rest (A) bedeutet, und der Verbindung der Formel (I), worin R₁ einen Rest (A) bedeutet und R₂ ein Wasserstoffatom bedeutet, mittels HPLC.

24. Verfahren nach Anspruch 23, wobei man die Chromatographie der Stufe o über eine ^{R}CHEM-ELUT-Säule durchführt, die von Prolabo vertrieben wird, die mit Wasser gesättigt ist, anschließend sukzessive mit Heptan, einem Gemisch aus Heptan-Ethylacetat (50/50 in Volumina), einem Gemisch aus Ethylacetat-n-Butanol (95/5; 90/10; 80/20; 50/50; 20/80, dann 0/100 in Volumina) und einem Gemisch aus n-Butanol-Wasser (98/2, dann 95/5 in Volumina) eluiert.

25. Verfahren nach Anspruch 24, wobei man die Chromatographie der Stufe p mit einem Gemisch aus Wasser-Ethanol (50/50 in Volumina) durchführt.

26. Verfahren nach Anspruch 24, wobei man die Chromatographie der Stufe q über eine YMC 180DS-AQ-Säule, vertrieben von AIT, mit einem Gemisch aus Wasser mit 0,1% Ameisensäure als Eluent durchführt.

27. Medikamente, dadurch **gekennzeichnet**, dass sie als Wirkstoff ein Gemisch I nach Anspruch 1 oder ein Gemisch II nach Anspruch 1 oder Natriumoxamat oder ein oder mehrere Verbindung(en) der Formel: 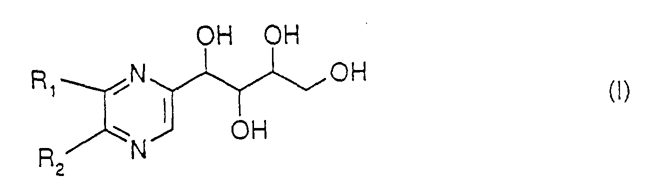 worin entweder R₁ ein Wasserstoffatom bedeutet und R₂ eine Kette der Formel:
-CH₂-CHOH-CHOH-CH₂OH (A)
-CHOH-CHOH-CHOH-CH₂OH (B)
bedeutet, oder R₁ eine Kette der Formel (A) bedeutet und R₂ ein Wasserstoffatom bedeutet, und ein oder mehrere Exzipientien umfassen.

28. Verwendung eines Gemisches I nach Anspruch 1 oder eines Gemisches II nach Anspruch 1 oder Natriumoxamat oder einer oder mehrerer Verbindungen der Formel: 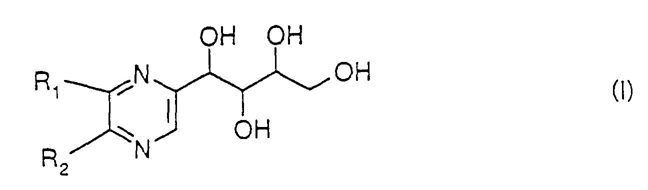 worin entweder R₁ ein Wasserstoffatom bedeutet und R₂ eine Kette der Formel:
-CH₂-CHOH-CHOH-CH₂OH (A)
-CHOH-CHOH-CHOH-CH₂OH (B)
bedeutet, oder R₁ eine Kette der Formel (A) bedeutet und R₂ ein Wasserstoffatom bedeutet, zur Herstellung eines Medikaments der Verhütung und Behandlung von Diabetes und Komplikationen von Diabetes.

## Claims

1. Mixtures free of polyphenol and sterol derivatives and which can be isolated by grinding Eugenia Jambolana Lamarck seeds, maceration of the powder with a lower aliphatic alcohol with the use of heat, filtration, recovery of the insoluble part no longer containing polyphenol and sterol compounds, treatment of the insoluble part with an ammoniacal solution, treatment of the ammoniacal mixture with a lower aliphatic alcohol with the use of heat, filtration, recovery of the insoluble matter and drying this insoluble matter which constitutes the mixture I and optionally treatment of the mixture I with a water-lower aliphatic alcohol solution, filtration, partial concentration of the filtrate, purification on nonpolar adsorbent resins, partial concentration, centrifugation, ultrafiltration and isolation of the mixture II.

2. Process for preparing the mixture I according to claim 1, characterized in that Eugenia Jambolana Lamarck seeds are dried, finely ground, screened and the powder obtained is subjected to the following treatment:
a - maceration, with stirring, in a lower aliphatic alcohol (1-4C), at a temperature of between 40 and 70°C,
b - filtration under vacuum and recovery of the insoluble matter,
c - maceration, with stirring, of the insoluble matter with a lower aliphatic alcohol (1-4C) at a temperature of between 40 and 70°C,
d - filtration under vacuum and removal of the alcoholic phases containing mainly the undesirable polyphenols and sterols,
e - taking up the insoluble matter in an ammoniacal solution at a temperature of between 10 and 30°C,
f - taking up the whole wet ammoniacal mass in a water-lower aliphatic alcohol (1-4C) solution, at a temperature of between 40 and 70°C,
g - filtration and removal of the alcoholic solution,
h - washing of the insoluble matter with a lower aliphatic alcohol (1-4C), filtration and removal of the alcoholic solution,
i - recovery of the insoluble matter and drying the mixture I.

3. Process according to claim 2, for which, in step a, the procedure is carried out by means of 2 to 10 litres of a lower aliphatic alcohol per 1 kg of screened powder.

4. Process according to either of claims 2 and 3, for which, in step a, 5 litres of ethanol with a titre of 93-95° Gay Lussac are used at 60°C for 1 hour.

5. Process according to one of claims 2 to 4, for which the filtration of steps b and d is carried out under a vacuum of 40 kPa.

6. Process according to one of claims 2 to 5, for which, in step c, the procedure is carried out by means of 2 to 10 litres of a lower aliphatic alcohol per 1 kg of starting screened powder.

7. Process according to one of claims 2 to 6, for which, in step c, 4 litres of ethanol with a titre of 93-95° Gay Lussac are used at a temperature of 60°C for 1 hour.

8. Process according to one of claims 2 to 7, for which, in step e, 750 to 1250 ml of an aqueous ammoniacal solution are used per 1 kg of starting screened powder.

9. Process according to one of claims 2 to 8, for which, in step e, per 1 kg of starting screened powder, 1 litre of aqueous ammoniacal solution containing 350 ml of 28% ammonium hydroxide is used and the procedure is carried out at a temperature close to 20°C for 10 to 30 hours.

10. Process according to one of claims 2 to 9, for which, in step f, the wet ammoniacal mass obtained from 1 kg of starting screened powder is taken up in 2 to 10 litres of a water-lower aliphatic alcohol solution.

11. Process according to one of claims 2 to 10, for which, in step f, the procedure is carried out in 5 litres of an ethanol-water mixture (75/25 by volume), at 60°C for 1 hour.

12. Process according to one of claims 2 to 11, for which, in step h, the washing is carried out with 500 to 1500 ml of a lower aliphatic alcohol per 1 kg of starting screened powder.

13. Process according to one of claims 2 to 12, for which, in step h, the washing is carried out with 1 litre of ethanol and the filtration is carried out on a cotton cloth and under a vacuum of about 80 kPa.

14. Process according to one of claims 2 to 12, for which, in step i, the drying is carried out in the open air and protected from light.

15. Process for preparing the mixture II according to claim 1, for which the mixture I according to claim 1 is subjected to the following operations:
j - treatment of the mixture I according to claim 1 by means of a water-lower aliphatic alcohol (1-4C) solution,
k - decantation and then, on the one hand, drawing off the top phase which is filtered to give the filtrate 1 and, on the other hand, treating the bottom phase with water and filtration to give the filtrate 2, pooling of the filtrates 1 and 2 and concentration to aqueous phase,
l - treatment with a nonpolar adsorbent resin and then filtration,
m - concentration of the filtrate, filtration and then ultrafiltration,
n - freeze-drying and isolation of the extract II.

16. Process according to claim 15, for which, in step j, 10 to 25 litres of the water-lower aliphatic alcohol solution (95/5 to 90/10 by volume) are used per 1 kg of the mixture I.

17. Process according to either of claims 15 and 16, for which, in step j, 18 litres of a water-ethanol solution (17.7-0.93 by volume) are used.

18. Process according to one of claims 15 to 17, for which, in step k, the top phase is filtered on a cotton cloth and 10 to 25 litres of water are added to the bottom phase per 1 kg of the mixture I and the mixture is filtered on sintered glass.

19. Process according to one of claims 15 to 18, for which, in step k, the concentration is generally carried out in a thermosiphon concentrator at a temperature of 35°C under a vacuum of 0.4 kPa.

20. Process according to one of claims 15 to 19, for which, in step 1, S861 resin or XAD-type resins marketed by Rhom and Haas are used and the mixture is filtered on sintered glass.

21. Process according to one of claims 15 to 20, for which, in step m, the concentration is carried out in a thermosiphon concentrator at a temperature of 35°C under a vacuum of 0.4 kPa.

22. Process according to one of claims 15 to 21, for which, in step m, 3 successive ultrafiltrations are carried out on 10 kDa, 3 kDa and 1 kDa cartridges.

23. Process for preparing sodium oxamate and the compounds of formula: 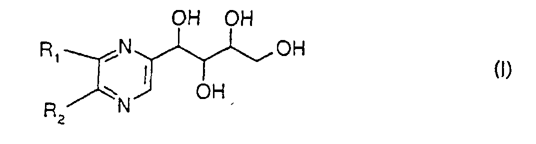 in which either R₁ represents a hydrogen atom and R₂ represents a chain of formula:
-CH₂-CHOH-CHOH-CH₂OH (A)
-CHOH-CHOH-CHOH-CH₂OH (B)
or R₁ represents a chain of formula (A) and R₂ represents a hydrogen atom for which the mixture II according to claim 1 is subjected to the following operations:
o - chromatography of the mixture II according to the invention on an infusorial earth column, recovery of the fractions containing the 4 products and pooling of these fractions into a single fraction,
p - chromatography of the fraction previously obtained on a sephadex® column in order to obtain sodium oxamate, the compound of formula (I) for which R₁ represents a hydrogen atom and R₂ represents a residue (B) and a mixture of the compound of formula (I) for which R₁ represents a hydrogen atom and R₂ represents a residue (A) and of the compound of formula (I) for which R₁ represents a residue (A) and R₂ represents a hydrogen atom,
q - optionally, chromatography of the mixture of the compound of formula (I) for which R₁ represents a hydrogen atom and R₂ represents a residue (A) and of the compound of formula (I) for which R₁ represents a residue (A) and R₂ represents a hydrogen atom by HPLC.

24. Process according to claim 23, for which the chromatography of step o is carried out on a CHEM ELUT® column marketed by Prolabo, saturated with water and then eluted successively with heptane, a heptane-ethyl acetate mixture (50/50 by volume), an ethyl acetate-n-butanol mixture (95/5; 90/10; 80/20; 50/50; 20/80 and then 0/100 by volume) and an n-butanol-water mixture (98/2 and then 95/5 by volume).

25. Process according to claim 24, for which the chromatography of step p is carried out by means of a water-ethanol mixture (50/50 by volume).

26. Process according to claim 24, for which the chromatography of step q is carried out on a YMC 180DS-AQ column marketed by AIT with, as eluent, a water containing 0.1% of formic acid mixture.

27. Medicaments characterized in that they contain, as active ingredient, a mixture I according to claim 1 or a mixture II according to claim 1 or sodium oxamate or one or more compounds of formula: 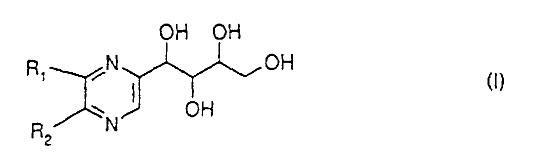 in which either R₁ represents a hydrogen atom and R₂ represents a chain of formula:
-CH₂-CHOH-CHOH-CH₂OH (A)
-CHOH-CHOH-CHOH-CH₂OH (B)
or R₁ represents a chain of formula (A) and R₂ represents a hydrogen atom and one or more excipients.

28. Use of a mixture I according to claim 1 or a mixture II according to claim 1 or sodium oxamate or one or more compounds of formula: 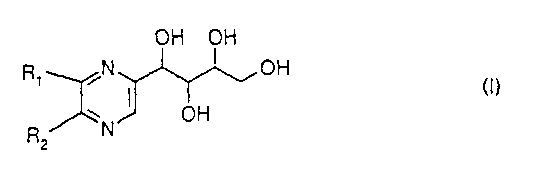 in which either R₁ represents a hydrogen atom and R₂ represents a chain of formula:
-CH₂-CHOH-CHOH-CH₂OH (A)
-CHOH-CHOH-CHOH-CH₂OH (B)
or R₁ represents a chain of formula (A) and R₂ represents a hydrogen atom for the preparation of a medicament for the prevention and the treatment of diabetes and of the complications of diabetes.
